# EUROPEAN PATENT SPECIFICATION

(11) **EP 2 579 884 B1**
(45) Date of publication and mention of the grant of the patent: **04.06.2014**
(21) Application number: 11726102.4
(22) Date of filing: 09.06.2011
(51) Int. Cl.: A61K 35/76, A61K 39/42, A61P 35/00

(54) **ANTI-TUMOR COMPOSITION**
ANTITUMORZUSAMMENSETZUNG
COMPOSITION ANTITUMORALE

(30) Priority: 14.06.2010 US 354361 P; 10.06.2010 EP 10165577
(43) Date of publication of application: 17.04.2013
(73) Proprietor: Intervet International B.V., 5831 AN Boxmeer (NL)
(72) Inventor: SCHRIER, Carla Christina, NL-5831 AN Boxmeer (NL); JAGT, Henricus Johannes Maria, NL- 5831Boxmeer AN (NL)
(74) Representative: Keus, Jacobus Albertus Ronald
(86) International application number: PCT/EP2011/059555
(87) International publication number: WO 2011/154476

(56) References cited:
- WO-A1-01/20989
- WO-A2-00/62735
- US-A1- 2009 208 495
- ISHIDA M ET AL: "Antigenic characterization of hemagglutinin-neuraminidase (HN) protein of avian paramyxoviruses by specific antisera to isolated HN subunits", ARCHIVES OF VIROLOGY, vol. 83, no. 3-4, 1985, pages 229-239, XP002608682,
- SCHIRRMACHER V ET AL: "Human tumor cell modification by virus infection: an efficient and safe way to produce cancer vaccine with pleiotropic immune stimulatory properties when using Newcastle disease virus", GENE THERAPY, vol. 6, no. 1, January 1999 (1999-01), pages 63-73, XP002608683,
- DAVIS J J ET AL: "Oncolytic virotherapy for cancer treatment: Challenges and solutions", JOURNAL OF GENE MEDICINE, WILEY, US, vol. 7, no. 11, 1 November 2005 (2005-11-01), pages 1380-1389, XP002531764, ISSN: 1099-498X, DOI: 10.1002/JGM.800 [retrieved on 2005-07-18]
- GARDNER AMANDA E ET AL: 'A conserved region in the F(2) subunit of paramyxovirus fusion proteins is involved in fusion regulation', August 2007, JOURNAL OF VIROLOGY AUG 2007, VOL. 81, NR. 15, PAGE(S) 8303 - 8314, ISSN 0022-538X

## Description

The present invention relates to pharmaceutical compositions comprising Avian Paramyxovirus (APMV) for use in the treatment of a tumor in a mammal.

Newcastle disease virus (NDV) is a member of the avian paramyxo viruses (APMV) that causes infection in a variety of birds. NDV belongs to the APMV 1. The disease is characterised by inflammation of the respiratory tract, the brain or the gastrointestinal tract.

It has been known for many decades, that Newcastle Disease virus has another and rather unexpected characteristic: for partially unknown reasons, it has certain anti-tumor effects in mammals. Therefore, next to the interest for vaccinating avian species, there is an increasing interest in the use of Newcastle disease and other paramyxo viruses in cancer therapies, including human cancer therapies.

When Newcastle Disease virus replicates in humans, generally spoken the virus does not behave virulent. The most well-known symptom in humans infected with NDV is a mild conjunctivitis. Such conjunctivitis is often experienced by veterinarians who are for the first time involved in vaccinating large amounts of chickens with live attenuated NDV.

However, for reasons not well understood, the pathogenicity for mammalian tumor cells is much higher, compared to the pathogenicity in non-tumor cells. It is estimated that ND replicates in cancer cells up to about 100.000 times better than in normal cells.

NDV is not the only APMV that has anti-tumor effects such as oncolytic effects. Currently, oncolytic strains of APMV 1, 3, 4, 5, 6, 7, 8, 9, Mapuerta virus and Fer-de-Lance virus are known, see e.g. US-Patent Application US2009/0208495.

There are two types of APMV: the lytic and the non-lytic strains. Both lytic and non-lytic strains can kill cancer cells, but lytic cells have a somewhat quicker mode of action. (Schirrmacher, V. et al., Int. J. Oncol. 2001 May; 18(5): 945-52). It is assumed that lytic strains damage the plasma membrane of infected cells, whereas non-lytic strains appear to interfere with the metabolism of the cell. Both lytic and non-lytic strains are thus toxic to tumor cells, albeit through different mechanisms. Therefore, in order to avoid confusion, both lytic and non-lytic strains will also be referred to further as cytotoxic strains. Since in the literature, lytic strains are also referred to as oncolytic strains, the wording lytic strain will refer here to oncolytic strains.

Both lytic and non-lytic NDV strains have been investigated for their use in combating cancer. This has led to the development of three different basic anti-tumor therapies:
1) Administration of a non-lytic or lytic APMV strain to a patient.
2) Administration of so-called oncolysates comprising plasma membrane fragments from *in vitro* APMV-infected cancer cells to a patient.
3) Administration of intact cancer cells infected with a non-lytic APMV strain to a patient.

The rationale behind the first approach is that the spread of the lytic virus strain and the subsequent replication of that strain will finally lead to infection of all tumor cells in the body. A disadvantageous consequence of this approach is, that an immune response will after some time be induced, which may neutralise the parent and/or progeny virus.

The rational behind the second and third approach is that tumor-specific antigens on the surface of tumor cells are better recognized when they are associated with viral antigens. The choice between the second and the third approach depends on which is supposed to provide a better response; plasma membranes or whole cells.

The disadvantage of virus-based anti tumor approaches 1, 2 and 3 is that an immune response against the APMV will after some time be induced, which may interfere with the parent and/or progeny virus and block infection of further cells.

This problem has been dealt with in several ways. Some approaches rely i.a. upon the introduction into the viral genome, of a heterologous gene that encodes a compound that interferes with the host's immune system.

Another approach is to give (very) high doses of virus several times a week, in order to either overcome the effect of induced antibodies or induce some kind of immune tolerance against the virus.

Other approaches try to avoid the necessity of second and further rounds of viral infection, through a very firm first attack. This can e.g. be done through the combined administration of NDV and some anti-tumor drug such as a cytotoxic or cytostatic compound. Other such approaches rely upon the introduction in NDV of genes encoding e.g. a full IgG antibody targeted against a tumor-specific antigen.

A shared disadvantage of these approaches is that each of them is more aggravating for the patient, when compared to the basic treatment with relatively low concentrations of NDV. Thus there is a need for alternative approaches to diminish the problems associated with antibody-induction.

It is an objective of the present invention to provide means to diminish the problems associated with antibody-induction without facing the disadvantages mentioned above.

In this respect, one embodiment of the present invention relates to a pharmaceutical composition comprising an Avian Paramyxovirus (APMV) for use in the treatment of a tumor in a mammal, wherein said treatment comprises the step of administering a cytotoxic amount of a first APMV to said mammal, followed by the step of administering a cytotoxic amount of a second APMV to that mammal within 2-56 weeks of the administration of the first APMV and wherein the second APMV has an HN protein that is immunologically different from that of the first APMV.

All APMV's carry a gene encoding Hemagglutinin/Neuraminidase (HN) activity and a gene encoding the Fusion (F) protein. The Hemagglutinin/IVeuraminidase is a strong inducer of a protective immune response, whereas the Fusion protein is also (albeit to a lesser) extent also involved in the induction of a protective immune response.

It was surprisingly found now that there is a strikingly low immunological cross-reactivity between the Hemagglutinin/Neuraminidase and the Fusion protein of the various members of the avian paramyxo viruses (APMV's). The immunological cross-reactivity is in some cases even practically non-existent. This unexpected finding opens new approaches that reduce, or preferably avoid the problems associated with immune-induction after a first administration of the APMV strain.

One way to reach this objective, is to administer a cytotoxic amount of a first APMV to a mammal, followed by the administration of a cytotoxic amount of a second APMV to that mammal within 2-56 weeks of said administration of the first APMV, taking care that the second APMV has an HN protein and a Fusion protein that is immunologically different from that of the first APMV.

Immunologically different in this respect means that the second HN protein and the Fusion protein are from an APMV that does not belong to the first APMV. Merely as an example, if the HN protein of the first APMV belongs to the APMV 1, the HN protein of the second APMV must belong to another APMV such as APMV 3 or APMV 5, in order to qualify as immunologically different.

By following this approach, the second APMV would be hampered less or even much less by a possible immune response against the first APMV, because of the (very) low immunological cross-reactivity between the different APMV's. This would allow for several rounds of virus administration over time. The advantage of such an approach is clear, even more when the tumor to be treated is a solid tumor. Especially in such cases it is not likely that all cells of the tumor mass are infected at the same moment. The core of the tumor would remain un-attacked at first. Those cells infected after a first virus administration would have to die and disappear before deeper cell layers in the tumor mass can be infected. By that time, immunity raised against the virus could well have removed the remaining virus and as a consequence these deeper cell layers would not be killed. A second round of APMV-administration, now however with a second (and where necessary a third or further) APMV-strain against which no immune response has been raised would solve this problem.

There are several ways to chose or select the first and second APMV. An easy way is to use one of the APMV's selected from the group consisting of APMV 1, 3, 4, 5, 6, 7, 8, 9, Mapuerta virus and Fer-de-Lance virus as a first APMV and another APMV of this group as a second APMV. Merely as an example: one could administer a cytotoxic amount of APMV 1 as a first APMV, followed by the administration of a cytotoxic amount of APMV 3 within 2-56 weeks after the first administration.

Another, more elaborate but elegant way to select the first and second APMV relies on the fact that, as said above, the main immune response against APMV's is directed against the HN of the virus, and albeit to a lesser extent to the F protein. By simply replacing the gene encoding the HN, and if desired the gene encoding the F protein, of a specific APMV by that of another APMV, one could use the same APMV backbone twice: one as the wild-type and a second time as a recombinant now carrying the (gene encoding the) HN and possibly also the F protein of another APMV instead of that of then wild-type. Merely as an example, one could use NDV as a first APMV and a recombinant NDV based upon the same NDV backbone but now carrying the (gene encoding the) HN and possibly the F protein of another APMV, e.g. APMV 3, as the second APMV, *instead* of the original NDV HN or Fusion protein. The second (the recombinant NDV) APMV would (much) less be hampered by the immune response raised against the first APMV because (in spite of the fact that the basis of the second APMV is NDV) the main immunogenic determinants of the second (recombinant NDV) would not be that of NDV but of another APMV, e.g. APMV 3.

The period of 2-56 weeks between the administration of the first and second APMV has the following rationale: some tumors are fast growing, whereas other tumors, or even metastasized tumor cells can be slowly growing or even be "dormant" for quite some time. Thus, depending on the characteristics of the tumor, it could be beneficial to give a second APMV earlier or later in time. In many cases, the period between the administration of the first and second APMV would be shorter, because the time of "dormancy" is less than 56 week. And moreover, one might want to avoid an risks of earlier outgrowth of cells. Thus, a preferred period would be between 2 and 28 weeks, more preferred between 2-20, 2-16, 2-12 or even 2-8 weeks in that order of preference.

This novel approach has the advantage over existing approaches, that it relies solely on the cytotoxic effects of APMV, thus in principle without the mandatory use of cytotoxic drugs or of compounds or regimes interfering with the immune system and immune response, as indicated above on which the known approaches are based.

An additional advantage of the present invention is the following: if after some time a dormant (or) metastasized tumor cell starts dividing after the patient has been treated with the composition according to the invention in two steps, the procedure can simply be repeated by administering a third APMV and if desired further APMV's.

Given the fact that a certain immune response is triggered against the F protein, preferably the second APMV has not only an HN protein that is immunologically different from that of the first APMV but also an F protein that is immunologically different from that of the first APMV.

It should be understood that, if e.g. an NDV backbone is used in both the first and second step, the HN and Fusion protein in the second, the recombinant, NDV should preferably originate from one and the same non-NDV APMV. As an example, if the first NDV is a wild-type NDV, the second NDV, the recombinant NDV should preferably carry both the HN and Fusions protein of e.g. APMV4 or of APMVS, and not the HN of APMV4 and the Fusion protein of APMVS.

Thus, a preferred embodiment of the present invention relates to pharmaceutical compositions according to the invention wherein the second APMV additionally has an F protein that is immunologically different from that of the first APMV.

The choice of viruses is quite extensive. APMV's suitable for anti-tumor therapy are known and have been known in the art for a long time. For instance, an overview of NDV strains used in human cancer studies comprises i.a. strain 73-T (Cassel WA, Garrett RE. Cancer 18: 863-8, 1965), Ulster (Bohle W, Schlag P, Liebrich W, et al. Cancer 66 (7): 1517-23, 1990.), MTH-68 (Csatary LK, Moss RW, Beuth J, et al. Anticancer Res 19 (1B): 635-8, 1999) (Csatary LK, Eckhardt S, Bukosza I, et al. Cancer Detect Prev 17 (6): 619-27, 1993.), Italien (Mallmann P. Hybridoma 12 (5): 559-66, 1993), Hickman (Wheelock EF, Dingle JH. N Engl J Med 271(13): 645-51, 1964), PV701 (Pecora AL, Rizvi N, Cohen GI, et al. J Clin Oncol 20 (9): 2251-66, 2002.), HUJ (Freeman AI, Zakay-Rones Z, Gomori JM, et al. Mol Ther 13 (1): 221-8, 2006) and LaSota (Liang W, Wang H, Sun TM, et al. World J Gastroenterol 9 (3): 495-8, 2003).

With regard to the route of administration, again, the existing knowledge in the art also gives the skilled person ample guidance. Merely as examples of the art, the following overview is provided:

In animal studies, NDV infection has been accomplished by i.a. intratumoral, intraperitoneal and intravenous route as reviewed in Schirrmacher V, Griesbach A, Ahlert T., Int J Oncol 18 (5): 945-52, 2001. NDV infection through the intramuscular or subcutaneous route has been reviewed by i.a.Heicappell R, Schirrmacher V, von Hoegen P, et al., Int J Cancer 37 (4): 569-77, 1986.

In human studies, in cases where patients have been infected with a lytic strain of NDV, intratumoral, intravenous or intramuscular injection has been used (Cassel WA, Garrett RE, Cancer 18: 863-8, 1965, Csatary LK, Moss RW, Beuth J, et al. Anticancer Res 19 (1B): 635-8, 1999, Pecora AL, Rizvi N, Cohen GI, et al., J Clin Oncol 20 (9): 2251-66, 2002, Csatary LK, Bakács T, JAMA 281 (17): 1588-9, 1999, Wheelock EF, Dingle JH, N Engl J Med 271(13): 645-51, 1964, Csatary LK., Lancet 2 (7728): 825, 1971.

Also used are the following routes: inhalation and direct injection into the colon (i.e., via a colostomy opening). (Csatary LK, Moss RW, Beuth J, et al. Anticancer Res 19 (1B): 635-8, 1999 Jan-Feb, Csatary LK, Eckhardt S, Bukosza I, et al.. Cancer Detect Prev 17 (6): 619-27, 1993).

Additionally, an extensive overview of the use of APMV's such as NDV in cancer therapy can be found in the Position Description Questionaire "Newcastle Disease Virus (PDQ®) Health Professional Version" of the National Cancer Institute.

A cytotoxic amount of APMV is the amount of virus necessary for the induction of cell death. Theoretically spoken, one APMV can infect and kill one cell. In a practical setting, however, one would administer an amount that is a multitude of the number of tumor-cells to be infected. Suitable amounts are e.g. given in Csatary LK, Eckhardt S, Bukosza I, et al.: Attenuated veterinary virus vaccine for the treatment of cancer. Cancer Detect Prev 17 (6): 619-27, 1993. Generally spoken, the very mild behavior of the infection in non-tumor cells in mammals allows for relatively high doses to be administered. Doses between the wide range of 10⁴ and 10¹² pfu would be acceptable doses. Doses in the range between 10⁵ and 10⁹ pfu would be preferable doses for most applications. The literature cited above gives ample guidance in this respect.

In case a Newcastle Disease infection is caused by a veto- or mesogenic NDV strain, the disease is notifiable in most Western countries. Therefore, if NDV strains are used in anti-tumor compositions, one would chose lentogenic strains, in order to avoid notification.

Of all APMV's, Newcastle Disease virus (NDV) is the most used virus. Therefore, there might be some preference regarding the use of this virus as either the first or the second APMV. Therefore, a more preferred embodiment of the invention relates to pharmaceutical compositions according to the invention wherein the first APMV is Newcastle Disease virus.

In case the second APMV is a recombinant APMV, thus carrying the (gene encoding the) HN protein and if desired also the F protein of another APMV instead of that APMV's wild type genes, the preferred APMV backbone for both the first and second APMV is NDV.

Another attractive APMV is APMV 3 as either the first or the second APMV. Therefore, another more preferred embodiment of the invention relates to pharmaceutical compositions according to the invention wherein the first APMV is APMV 3.

Even more preferred are pharmaceutical compositions according to the invention wherein the first APMV is Newcastle Disease virus and the second APMV is APMV 3, or vice versa. Therefore, an even more preferred embodiment of the invention relates to pharmaceutical compositions according to the invention wherein the first APMV is Newcastle Disease virus and the second APMV is APMV 3.

Another such even more preferred embodiment of the invention relates to pharmaceutical compositions according to the invention wherein the first APMV is APMV 3 and the second APMV is Newcastle Disease virus.

As mentioned above, lytic APMV's act faster in the sense that they kill the cell quicker, if compared to non-lytic APMV's. Therefore, preferably one or more of the APMV's should be a lytic APMV.

Thus, a still even more preferred embodiment of the invention relates to pharmaceutical compositions according to the invention wherein at least the first or the second APMV is lytic.

A most preferred form of this embodiment relates to pharmaceutical compositions according to the invention wherein both the first and the second APMV are lytic.

Especially in the developed countries, there is an increasing interest in, and care for felines and canines that suffer from cancer. Like in humans, an increased life span increases the cancer rate in these animals. And the pharmaceutical compositions according to the invention have been shown to work very well in felines and canines.

Thus, another form of this embodiment relates to pharmaceutical compositions according to the invention for use in companion animals, such as equine, ferret, feline and canine species. Preferably such compositions would be for use in equine and canine species, more preferable for use in canine species.

As indicated above, the pharmaceutical compositions, when used as such, have significant advantages over the known anti-cancer approaches. Nevertheless, there may still be reasons to combine the pharmaceutical compositions according to the invention with any anti-tumor agent. An extensive list of such anti-tumor agents is given e.g. in US-Patent Application US2009/0208495.

Thus, another form of the embodiment relates to pharmaceutical compositions according to the invention wherein during the administration of at least the first or the second APMV, an amount of anti-tumor agent such as a cytotoxic drug is co-administered.

The first and/or second APMV may be a recombinant APMV additionally carry a heterologous gene e.g. encoding an enzyme for conversion of a pro-drug, or a binding protein. Such a binding protein could e.g. be an antibody. Another example of such gene could be a gene encoding a fusion protein that carries an immunoglobulin domain, as described in WO 2006/050984

Thus, another form of the embodiment relates to pharmaceutical compositions according to the invention wherein at least the first or the second APMV is a recombinant APMV carrying an additional gene.

The pharmaceutical composition according to the invention should in principle comprise the APMV in a pharmaceutically acceptable carrier, in order to allow for the administration of the APMV. The nature of the carrier depends i.a. upon the route of administration. If the administration route is through inhalation, the carrier could be as simple as sterile water, a physiological salt solution or a buffer. If injection is the preferred route, the carrier should preferably be isotonic and have pH restrictions that make it suitable for injection. Such carriers however are extensively known in the art.

Examples of pharmaceutically acceptable carriers useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer). Especially when such stabilisers are added to the vaccine, the vaccine is very suitable for freeze-drying. Freeze-drying is a very suitable method to prevent APMV from inactivation. Therefore, in a more preferred form, pharmaceutical composition according to the invention are in a freeze-dried form.

Recombinant APMV's carrying a heterologous gene can i.a. be prepared by the well-known reverse genetics technique described for many non-segmented negative-stranded RNA-viruses including APMV's. See e.g. Conzelmann, J. Gen. Virol. 77: 381-389 (1996), Conzelmann, Ann. Rev. Genet. 32, 123-162 (1998), Palese et al., Proc. Natl. Acad. Sci. 93: 11354-11358 (1996), Peeters et al., J. Virology 73: 5001-5009 (1999), Römer-Oberdörfer et al, J. Gen virol. 80: 2987-2995 (1999).

### EXAMPLES:

### Example 1

### SAFETY AND REPLICATION OF DIFFERENT LENTOGENIC APMV STRAINS IN DOGS

### 1 INTRODUCTION

1.1 The aim of this experiment was to asses whether lentogenic avian paramyxo viruses (APMV) NDV Clone 30, NDV Ulster and APMV 3, are safe and replicate in dogs.

### 2 MATERIALS AND METHODS

### 2.1 Short outline of the experiment

Three (3) groups of Beagle dogs, 2 animals per group, were inoculated via the oral, nasal, oculo and s.c. route with lentogenic live NDV Clone 30, NDV Ulster or APMV 3 as indicated in *Table 1 "Grouping and dosing".* Four dogs, divided in 3 groups (1-1-2), were used as contact controls (sentinels). At 3-6-9-13 and 15 days post inoculation oral, ocular and rectal swabs were taken. Nasal swabs were taken only at 3 and 6 days post inoculation. Blood samples were taken every week up to 8 weeks post inoculation. At 6 weeks post first inoculation dogs were inoculated for the second time via the oral, nasal, oculo and s.c. route with either NDV Clone 30 or APMV 3 (see *Table 1 "Grouping and dosing"*). At 3 and 6 days post 2^{nd} inoculation oral, nasal, ocular and rectal swabs were taken. Swabs are planned to be used for virus re-isolation. At 3 and 6 days post 1^{st} and 2^{nd} inoculation urine samples were taken via the urine bladder (if necessary a diuretic was used). During a period of 14 days post each inoculation the temperature of each dog was measured using the implanted chip. Dogs were observed daily for the occurrence of clinical signs of disease or other abnormalities. Eight weeks after the 1^{st} inoculation, i.e. 2 weeks post 2^{nd} inoculation, dogs were euthanized and macroscopically investigated. Samples for histology were taken from pancreas, spleen, liver, kidney, brains, heart, trachea, lungs and inguinal lymph node.

### 2.2 Strains used:

| | |
|---|---|
| Live NDV Ulster: | - 9.7 log10 EID₅₀ per ml |
| Live NDV Clone 30: | - 9.5 log10 EID₅₀ per ml |
| Live APMV 3: | - 8.7 log10 EID₅₀ per ml |

**Table 1: Grouping and dosing**

| | | | | **inoculation protocol (volume in ml)** | | | |
|---|---|---|---|---|---|---|---|
| **group** | **N** | **1^{st} inoc.** | **2^{nd} inoc.** | **oral** | **oculo: left & right** | ***intranasal: left & right** | **s.c. (left flank)** |
| **1** | **2** | **NDV Ulster** | **NDV. Clone 30** | **5.0** | **1x 0.25** | **1 puff** | **1.0** |
| **2** | **1** | **--** | **--** | **--** | **--** | **--** | **--** |
| **3** | **2** | **NDV Clone 30** | **NDV Clone 30** | **5.0** | **1 x 0.25** | **1 puff** | **1.0** |
| **4** | **2** | **--** | **--** | **--** | **--** | **--** | **--** |
| **5** | **2** | **APMV3** | **APMV3** | **5.0** | **1x 0.25** | **1 puff** | **1.0** |
| **6** | **1** | **--** | **--** | **--** | **--** | **--** | **--** |

| | | | | | | | |
|---|---|---|---|---|---|---|---|
| *The intranasal inoculation will be performed using a manual nebulizer. 1 puff equals ~ 72 µl. | | | | | | | |

### 2.3 Inoculations

Dogs were inoculated with live APMV's according to Table 1 at T=0. Six weeks after the 1^{st} inoculation animals received the 2^{nd} inoculation as indicated in Table 1.

### 2.4 Blood samples

Blood samples for serology were taken from all animals every week up to 8 weeks. Blood samples (coagulated and heparinized) after the 1^{st} and 2^{nd} inoculation were taken at 6 days in stead of 7 days. Blood sampling (at least 4-5 ml per dog) was done via the Jugular vein according to SOP 5619.074 and before inoculation. Blood samples (coagulated) were used to determine the HI and IFT titers.

### 2.5 HI-assay

Serum levels of NDV-specific antibodies at T=4, T=6 and T=8 weeks were determined by a haemagglutination-inhibition (HI) assay. Serial two-fold dilutions of sera were prepared in microtiter plates and mixed with an equal volume containing 8 haemagglutinating units/50 µl NDV antigen. Titres are expressed as the reciprocal of the highest dilution that gives complete inhibition of haemagglutination of chicken red blood cells (1% (v/v) in buffered saline). Samples were regarded positive for inhibition of haemagglutination at a dilution ≥1:2. Serum of each inoculated dog was tested for cross reactivity against all 3 APMV's.

### 2.6 IFT-assay

Serum levels of NDV-specific antibodies at T=4, T=6 and T=8 weeks were also determined by an immunofluorescense test (IFT). Microtiter plates were 'coated' overnight with 100 µl/well 1.5x10⁶/ml chick embryo fibroblasts (CEF) in RPMI 1640+standard antibiotics mixture+5% FCS at 37°C/5% CO₂. After 24 hrs the medium was replaced with 100 µl 1:100 in RPMI 1640+standard antibiotics mixture medium diluted APMV virus (NDV Clone 30, NDV Ulster or APMV 3). After 24 hrs the plates were emptied and the infected CEFs were fixated with 100 µl/well iced 96% ethanol (-70°C) during 30 minutes. Serial two-fold dilutions of dog sera were prepared in microtiter plates (next to several NDV positive and NDV negative chicken sera), added to the (washed) coated plates and incubated at 37°C for 1 hr. Plates were subsequently washed (3x) and incubated with 1:20 diluted FITC-labeled Goat-anti-Dog IgG (H+L) or Goat-anti-Chicken IgG (H+L) polyclonal antibodies. After 1 hr at 37°C the plates were washed (3x) and 20 µl PBS/glycerol (1:1) was added to each well. Titres are expressed as the reciprocal of the highest dilution that gives a specific fluorescent signal. Titres of ≥12 are expressed as 13 (log2). Serum of each inoculated dog was tested for cross reactivity against all 3 APMV's.

### 2.7 Swabs

At T=3-6-9-13 and 15 days post 1^{st} inoculation oral, ocular and rectal swabs were taken. A nasal swab was only taken at 3 and 6 days post 1^{st} and 2^{nd} inoculation. At 3 and 6 days post 2^{nd} inoculation oral, nasal, ocular and rectal swabs were taken. Swabs were collected in 2.5 ml of Tryptose 2.5% to which 1000 U/1000µg per ml Pen/Strep was added (storage at - 70°C).

### 2.8 Urine

At 3 and 6 days post 1^{st} and 2^{nd} inoculation a urine sample was taken via the urine bladder (if necessary a diuretic was used).

### 2.9 Virus re-isolation

Re-isolation of virus was performed by inoculation of 10-day-old embryonated eggs (N=8) with 0.1 ml of undiluted sample material. Following incubation for 4-6 days the allantoic fluid from all eggs was tested for HA activity according to the method of Spaerman and Kaerber (*In:* B. Bibrack and G. Whittmann, Editors, Virologische arbeitsmethoden, Fisher Verlag, Stuttgart (1974), pp. 37-39*).*

### 2.10 Body temperature

On the day of inoculation and during a period of 14 days post each inoculation the temperature of each dog was measured using the implanted chip.

### 2.11 Observation

Animals were observed daily for the presence of clinical signs of disease or other abnormalities.

### 2.12 Histology and pathology

At the end of the experiment, *i.e.* 8 weeks after the 1^{st} inoculation / 2 weeks after the 2^{nd} inoculation, all dogs were euthanized and macroscopically investigated. Samples for histology were taken from pancreas, spleen, liver, kidney, brains, heart, trachea, lungs and inguinal lymph node.

### RESULTS

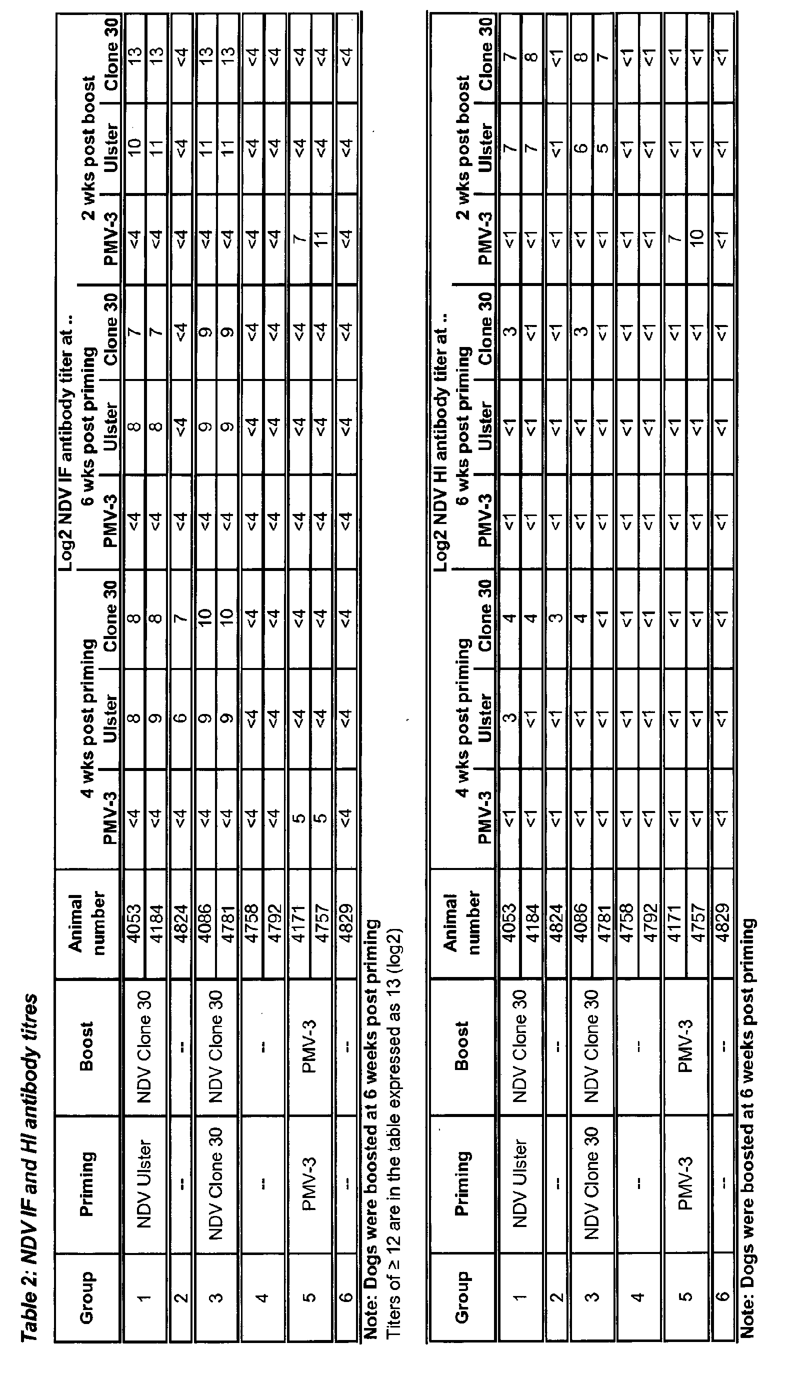

### 3.1 NDV IF and HI antibody titres

NDV IF antibody titers: 4 weeks post 1^{st} inoculation antibodies against the inoculated APMV could be detected in Gr1, Gr3 and Gr5 indicating that these APMV strains replicated in the dog and induced antibodies. From the data it is also clear that antibodies raised against NDV Ulster cross react with NDV Clone 30 and *vice versa.* This is not the case for the APMV 3 specific antibodies which do not cross react with NDV Ulster nor NDV Clone 30. At 6 weeks post 1^{st} inoculation antibody titers are comparable with the 4 weeks data. Interestingly, 2 weeks post 2^{nd} inoculation (dogs received a 2^{nd} inoculation at T=6 weeks) in all groups an increase in the antibody titer could be detected. This indicates that the second inoculation with live virus induces a booster effect resulting in an increased antibody titer due to replication of the virus.

NDV HI antibody titers: From these data it is even more clear that the second inoculation with live virus induced a strong booster effect which resulted in high antibody titers. Especially in Cr1 and Gr3 it is clear that the antibody titer dropped at T=6 weeks when compared with T=4 weeks, and that 2 weeks after the 2^{nd} inoculation the antibody titers are increased again and are higher when compared to T=4 weeks. Also these data indicate that the virus replicates in the dog.

### 3.2 Swabs and urine samples

The oral, ocular and rectal swabs and the urine samples from all dogs which were collected at 3 and 6 days post first inoculation were used for virus re-isolation. All samples were scored negative.

### 3.3 Body temperature and observation

On the day of inoculation and during a period of 14 days post each inoculation the temperature of each dog was measured using the implanted chip. No remarkable temperature shifts were noted. During the experiment no remarkable observations were made with regard to the presence of clinical signs of disease or other abnormalities in the dogs.

### 3.4 Histology and pathology

At the end of the experiment all dogs were euthanized and macroscopically investigated. The macroscopic observations revealed that in 1 dog from Gr5 a large white spot on the spleen, of 3 mm thick was noted. This corresponded microscopically with a focal moderate acute sub capsular haematoma. Splenic haematomas in dogs are mostly of traumatic origin. All other animals presented no macroscopic lesions at necropsy. Samples for histology were taken from pancreas, spleen, liver, kidney, brains, heart, trachea, lungs and inguinal lymph node. It was concluded that the inoculation of lentogenic avian paramyxo viruses in the dog via the nasal, ocular and oral route, did result in inflammatory lesions in the lungs especially in dogs inoculated with NDV Clone 30 and NDV Ulster. In one dog inoculated with APMV 3 a pancreatic inflammatory lesion and a severe haemorrhage was noted.

### Conclusion:

1) NDV Clone 30, NDV Ulster and APMV 3 are infectious to dogs and capable of replicating in dogs.
2) No clinical signs were found in the dogs, and no virus could be re-isolated. This shows that the use of such viruses in dogs is safe.
3) No cross-immunity exists between the NDV-strains and the APMV 3 strain.

### Example 2

Growth of APMV 3 on a human tumor cell line

### Cells used for cell culture:

Human colon cancer cell line CL 188

### Culture medium used:

### CL 188 :

RPMI 1640 + 10% FBS + 1x standard antibiotics mixture + L-glutamin + 2µg/ml amphotericin B (growth medium)
RPMI 1640 + 2% FBS + 1x standard antibiotics mixture + 2µg/ml amphotericin B (maintenance medium).

### Virus strain:

Avian paramyxovirus type 3
9.7 log10 EID₅₀/ml

### Egg source:

L11103 10-day old embryonated eggs
L11203 11-day old embryonated eggs

### Titration and HA-test:

- Tryptose 2.5%
- Pen-Strep
- 5% chicken red blood cells
- 0.1M PBS

### Inoculation of cells with APMV 3

1 flask of every cell line/ culture medium was harvested three days after passage.

The cells were counted to determine the dilution factor for viral inoculation with a MOI of 0.1 (CL 188).

Dilutions were made in the appropriate culture medium.

Inoculation of the cells was done as follows: the culture medium of adherent cells (CL 188) was removed. Next, 1 ml of virus was added to the cells. The cells were incubated for 1 hour at 37°C after which 4 ml of fresh culture medium was added to the cells + virus.

Cells were incubated at 37°C for another 4 days.

After inoculation the remaining diluted virus was stored at -20°C.

After 4 days the culture flasks were freeze-thawed at -20°C for three times.

After the last thaw the cells were transferred to a 15 ml tube and spun down for 5 minutes at 200 x G.

The supernatant was collected and stored in cryo tubes at -70°C.

### Determining viral growth by titration on eggs:

Titrations were performed on the harvest and inoculate from virus grown on CL 188 cells with 2% FBS.

Samples were diluted in 10-fold in tryptose until dilution 10⁻⁷.

10 10-day old embryonated eggs were injected with 0.2 ml of diluted sample (dilution 10⁻² /10⁻⁷). Eggs were incubated for four days at 37°C.

Titers were determined by HA-test.

Titrations were repeated on the harvest and inoculate from virus grown on CL 188 cells with 2% FBS.

Samples were diluted in 10-fold in tryptose until dilution 10⁻⁴ (CL 188 inoculate) or 10⁻⁵ (CL 188 harvest).

11 day old embryonated eggs were injected with 0.2 ml of diluted sample (dilution 10⁻¹/10⁻⁴ for CL 188 inoculate or dilution 10⁻²/10⁻⁵ for CL 188 harvest)

Eggs were incubated for three days at 37°C.

Titers (log10) were determined by HA-test.

### RESULTS

After 4 days incubation a clear CPE (dead cells) was visible on the CL 188 cells infected with APMV 3 virus

### Titration results

| **Sample** | **1st titration** | **2^{nd} titration** |
|---|---|---|
| CL 188 APMV 3 inoculate | -* | - |
| CL 188 APMV 3 harvest | log base 10 | log base 10 |
| | 4.8 EID₅₀/ml | 4.9 EID₅₀/ml |

| | | |
|---|---|---|
| * -= no titer found. | | |

Conclusion: It was shown that APMV 3 virus grows well on human colon cancer cell line CL188 with visible CPE.

## Claims

1. Pharmaceutical composition comprising an Avian Paramyxovirus (APMV) for use in the treatment of a tumor in a mammal, wherein said treatment comprises the step of administering a cytotoxic amount of a first APMV to said mammal, followed by the step of administering a cytotoxic amount of a second APMV to said mammal within 2-56 weeks of said administration of the first APMV and wherein said second APMV has an HN protein that is immunologically different from that of the first APMV, and
wherein said second APMV additionally has an F protein that is immunologically different from that of the first APMV.

2. Pharmaceutical composition for use according to claim 1 or 2, wherein the first APMV is a Newcastle Disease virus.

3. Pharmaceutical composition for use according to claim 1 or 2, wherein the first APMV is an APMV 3.

4. Pharmaceutical composition for use according to claim 3, wherein the first APMV is a Newcastle Disease virus and the second APMV is an APMV 3.

5. Pharmaceutical composition for use according to claim 4, wherein the first APMV is an APMV 3 and the second APMV is a Newcastle Disease virus.

6. Pharmaceutical composition for use according to any of claims 1-6, **characterised in that** at least the first or the second APMV is lytic.

7. Pharmaceutical composition for use according to claim 7, **characterised in that** both the first and the second APMV are lytic.

8. Pharmaceutical composition for use according to any of claims 1-8, **characterised in that** the mammal is of an equine, canine or feline species.

9. Pharmaceutical composition for use according to any of claims 1-9, **characterised in that** during the administration of at least the first or the second APMV, an amount of anti-tumor agent is co-administered.

10. Pharmaceutical composition for use according to any of claims 1-10, **characterised in that** at least the first or the second APMV is a recombinant APMV carrying an additional gene.

## Patentansprüche

1. Pharmazeutische Zusammensetzung, umfassend ein Vogel-Paramyxovirus (APMV) zur Verwendung bei der Behandlung eines Tumors in einem Säugetier, wobei die Behandlung den Schritt umfasst, bei dem eine cytotoxische Menge eines ersten APMV an das Säugetier verabreicht wird, und danach den Schritt, bei dem innerhalb von 2 bis 56 Wochen der Verabreichung des ersten APMV eine cytotoxische Menge eines zweiten APMV an das Säugetier verabreicht wird, und wobei das zweite APMV ein HN-Protein aufweist, das sich von dem des ersten APMV immunologisch unterscheidet, und
wobei das zweite APMV zudem ein F-Protein aufweist, das sich von dem des ersten APMV immunologisch unterscheidet.

2. Pharmazeutische Zusammensetzung zur Verwendung nach Anspruch 1 oder 2, wobei das erste APMV ein Newcastle Disease Virus ist.

3. Pharmazeutische Zusammensetzung zur Verwendung nach Anspruch 1 oder 2, wobei das erste APMV ein APMV 3 ist.

4. Pharmazeutische Zusammensetzung zur Verwendung nach Anspruch 3, wobei das erste APMV ein Newcastle Disease Virus ist, und das zweite APMV ein APMV 3 ist.

5. Pharmazeutische Zusammensetzung zur Verwendung nach Anspruch 4, wobei das erste APMV ein APMV 3 ist und das zweite APMV ein Newcastle Disease Virus ist.

6. Pharmazeutische Zusammensetzung zur Verwendung nach einem der Ansprüche 1 bis 6, **dadurch gekennzeichnet, dass** mindestens das erste oder das zweite APMV lytisch ist.

7. Pharmazeutische Zusammensetzung zur Verwendung nach Anspruch 7, **dadurch gekennzeichnet, dass** sowohl das erste als auch das zweite APMV lytisch sind.

8. Pharmazeutische Zusammensetzung zur Verwendung nach einem der Ansprüche 1 bis 8, **dadurch gekennzeichnet, dass** das Säugetier eine Pferde-, Hunde- oder Katzenart ist.

9. Pharmazeutische Zusammensetzung zur Verwendung nach einem der Ansprüche 1 bis 9, **dadurch gekennzeichnet, dass** während der Verabreichung von mindestens dem ersten oder zweiten APMV gleichzeitig eine Menge Antitumormittel verabreicht wird.

10. Pharmazeutische Zusammensetzung zur Verwendung nach einem der Ansprüche 1 bis 10 **dadurch gekennzeichnet, dass** mindestens das erste oder zweite APMV ein rekombinantes APMV ist, das ein zusätzliches Gen trägt.

## Revendications

1. Composition pharmaceutique comprenant un Paramyxovirus aviaire (APMV) destinée à être utilisée dans le traitement d'une tumeur chez un mammifère, dans lequel ledit traitement comprend l'étape consistant à administrer une quantité cytotoxique d'un premier APMV au dit mammifère, suivie de l'étape consistant à administrer une quantité cytotoxique d'un deuxième APMV au dit mammifère dans les 2 à 56 semaines suivant ladite administration du premier APMV et dans lequel ledit deuxième APMV a une protéine HN qui est immunologiquement différente de celle du premier APMV, et dans lequel ledit deuxième APMV a en plus une protéine F qui est immunologiquement différente de celle du premier APMV.

2. Composition pharmaceutique destinée à être utilisée selon la revendication 1 ou 2, dans laquelle le premier APMV est un virus de la maladie de Newcastle.

3. Composition pharmaceutique destinée à être utilisée selon la revendication 1 ou 2, dans laquelle le premier APMV est un APMV 3.

4. Composition pharmaceutique destinée à être utilisée selon la revendication 3, dans laquelle le premier APMV est un virus de la maladie de Newcastle et le deuxième APMV est un APMV 3.

5. Composition pharmaceutique destinée à être utilisée selon la revendication 4, dans laquelle le premier APMV est un APMV 3 et le deuxième APMV est un virus de la maladie de Newcastle.

6. Composition pharmaceutique destinée à être utilisée selon l'une quelconque des revendications 1 à 6, **caractérisée en ce qu'**au moins le premier ou le deuxième APMV est lytique.

7. Composition pharmaceutique destinée à être utilisée selon la revendication 7, **caractérisée en ce que** le premier et le deuxième APMV sont lytiques tous les deux.

8. Composition pharmaceutique destinée à être utilisée selon l'une quelconque des revendications 1 à 8, **caractérisée en ce que** le mammifère est d'une espèce équine, canine ou féline.

9. Composition pharmaceutique destinée à être utilisée selon l'une quelconque des revendications 1 à 9, **caractérisée en ce que**, lors de l'administration du au moins premier ou deuxième APMV, on administre conjointement une quantité d'agent anti-tumoral.

10. Composition pharmaceutique destinée à être utilisée selon l'une quelconque des revendications 1 à 10, **caractérisée en ce qu'**au moins le premier ou le deuxième APMV est un APMV recombinant, qui transporte un gène supplémentaire.
